# EUROPEAN PATENT APPLICATION

(11) **EP 4 696 697 A1**
(43) Date of publication of application: **18.02.2026**
(21) Application number: 23933156.4
(22) Date of filing: 17.07.2023
(51) Int. Cl.: C07H 19/20, C07H 1/06, C12P 19/32

(54) **METHOD FOR MANUFACTURING DISODIUM-5'-GUANYLATE CRYSTALS**

(30) Priority: 12.04.2023 KR 20230048102
(71) Applicant: CJ Cheiljedang Corporation, Seoul 04560 (KR)
(72) Inventor: LIM, Hwayeon, Seoul 04560 (KR); KIM, Min Jong, Seoul 04560 (KR); LEE, Joohang, Seoul 04560 (KR); PARK, Young Soo, Seoul 04560 (KR); LEE, Seung-je, Seoul 04560 (KR); YU, Jae Hun, Seoul 04560 (KR)
(74) Representative: Meissner Bolte Partnerschaft mbB
(86) International application number: PCT/KR2023/010158
(87) International publication number: WO 2024/214870

(57) **Abstract**

The present disclosure relates to a method for preparing disodium-5'-guanylate crystals, comprising: a mixing step of adding an aqueous sodium chloride solution to an aqueous disodium-5'-guanylate solution; a concentration step of concentrating the mixed solution of the aqueous disodium-5'-guanylate solution and the aqueous sodium chloride solution and precipitating amorphous crystals of disodium-5'-guanylate; and a transition step of adding seeds to the mixed solution of the aqueous disodium-5'-guanylate solution and the aqueous sodium chloride solution to transform the amorphous crystals of disodium-5'-guanylate into columnar crystals. According to the present disclosure, the disodium-5'-guanylate crystals can be produced in high yield by an environmentally friendly method without the use of organic solvents.

## Description

### [Technical Field]

The present disclosure relates to a method for preparing disodium-5'-guanylate crystals in an environmentally friendly manner without the use of organic solvents.

### [Background Art]

A generally known solvent crystallization method utilizes hydrophilic organic solvents in the preparation of fermentation-derived disodium-5'-guanylate. This process has the disadvantage of increasing the costs of auxiliary materials and utility. In addition, the crystallization method using hydrophilic organic solvents deviates from ESG principles, thus, process improvement is required in accordance with environmental changes.

### [Disclosure]

### [Technical Problem]

It is one object of the present disclosure to provide a technique which enables the preparation of disodium-5'-guanylate crystals with high yield without the use of organic solvents.

### [Technical Solution]

The present disclosure, which aims to solve the above technical problem, has the following structure and characteristics:
One aspect of the present disclosure provides a method for preparing disodium-5'-guanylate crystals, including:
a mixing step of adding an aqueous sodium chloride solution to an aqueous disodium-5'-guanylate solution;
a concentration step of concentrating the mixed solution of the aqueous disodium-5'-guanylate solution and the aqueous sodium chloride solution, and precipitating amorphous crystals of disodium-5'-guanylate; and
a transition step of adding seeds to the mixed solution of the aqueous disodium-5'-guanylate solution and the aqueous sodium chloride solution to transform the amorphous crystals of disodium-5'-guanylate into columnar crystals.

In one embodiment, in the transition step, the formation of amorphous crystals of disodium-5'-guanylate and the transition of amorphous crystals of disodium-5'-guanylate into columnar crystals may be performed continuously.

In another embodiment, the amorphous crystals of disodium-5'-guanylate may include a hydrate of disodium-5'-guanylate.

In still another embodiment, the concentration step may be performed such that the disodium-5'-guanylate is concentrated to a concentration of 400 g/L to 600 g/L.

In yet another embodiment, the preparation method may further include adjusting the pH of the aqueous disodium-5'-guanylate solution to 8 to 10 before performing the mixing step.

In even another embodiment, the pH adjustment of the aqueous disodium-5'-guanylate solution may be performed by adding sodium salt.

In further another embodiment, in the mixing step, sodium chloride may be added at a ratio of 30% to 80% by weight of disodium-5'-guanylate.

In still further another embodiment, in the transition step, the seed may be added at a ratio of 0.5% to 50% by weight of the disodium-5'-guanylate.

In still further another embodiment, in the transition step, the seed may be added when the concentration of disodium chloride in the mixed solution is the concentration of disodium-5'-guanylate is about 100 g/L to about 130 g/L.

Another aspect of the present disclosure provides a fermentation product which includes disodium-5'-guanylate crystals in columnar form, wherein the concentration of chlorine ions (Cl⁻) in the disodium-5'-guanylate crystals is 500 ppm to 20,000 ppm.

In one embodiment, the fermentation product may further include disodium 5'-inosinate crystals, wherein the concentration of chlorine ions (Cl⁻) in the mixture of disodium-5'-guanylate crystals and disodium-5'-inosinate crystals is 500 ppm to 20,000 ppm.

In another embodiment, the disodium-5'-guanylate crystals may be provided in the form of disodium-5'-guanylate heptahydrate or disodium-5'-guanylate tetrahydrate.

### [Advantageous Effects]

The method for preparing disodium-5'-guanylate crystals according to one aspect of the present disclosure is environmentally friendly as disodium-5'-guanylate crystals are prepared using an aqueous solvent without an organic solvent. Additionally, the yield of disodium-5'-guanylate crystals may be increased by adjusting the process factors so that disodium-5'-guanylate is prepared in the form of amorphous crystals and then undergoes a phase transition to columnar crystals.

Further, according to one aspect of the present disclosure, since 500 ppm to 20,000 ppm of chlorine ions are contained, disodium-5'-guanylate crystals having high storage and distribution stability can be provided.

### [Brief Description of Drawings]

FIG. 1 is a flow chart showing the method for preparing disodium-5'-guanylate according to the present disclosure.
FIG. 2 is a flowchart showing the method for preparing disodium-5'-guanylate crystals according to one aspect of the present disclosure.
FIG. 3 shows the results of XRD analysis of disodium-5'-guanylate crystals obtained according to Example 2.
FIG. 4 is an image of disodium-5'-guanylate crystals obtained according to Example 4 observed under a microscope.
FIG. 5 shows the particle size analysis result of disodium-5'-guanylate crystals obtained according to Example 4.

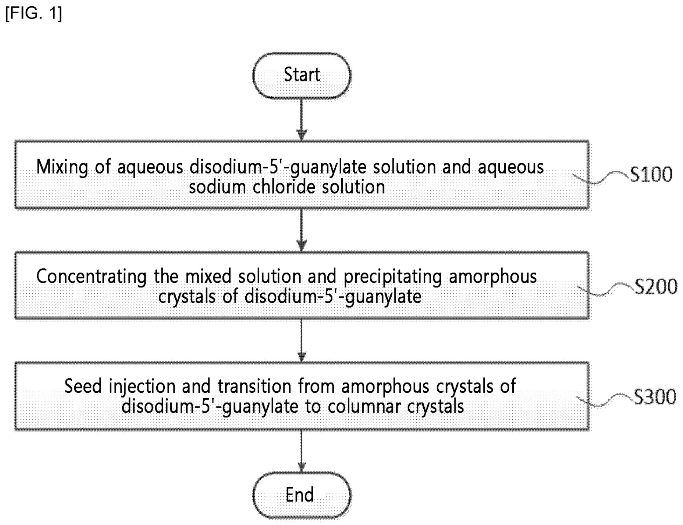

FIG. 1 is a flow chart showing the method for preparing disodium-5'-guanylate according to the present disclosure.

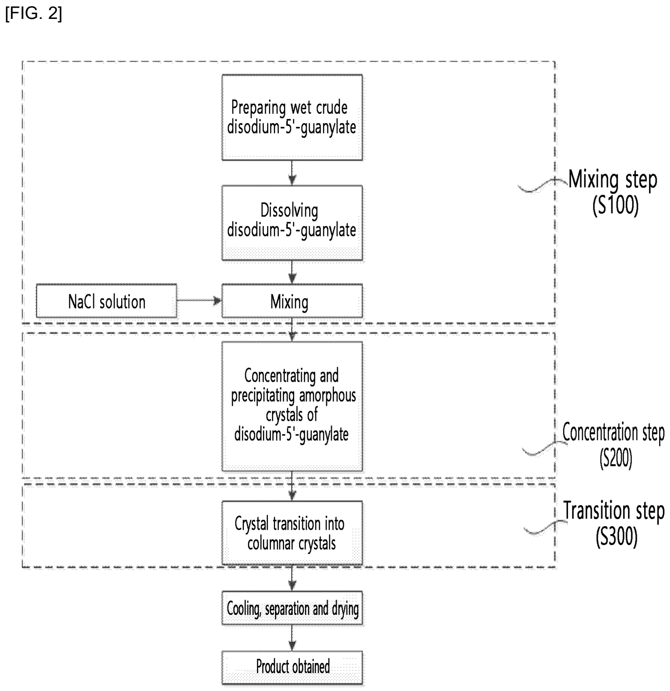

FIG. 2 is a flowchart showing the method for preparing disodium-5'-guanylate crystals according to one aspect of the present disclosure.

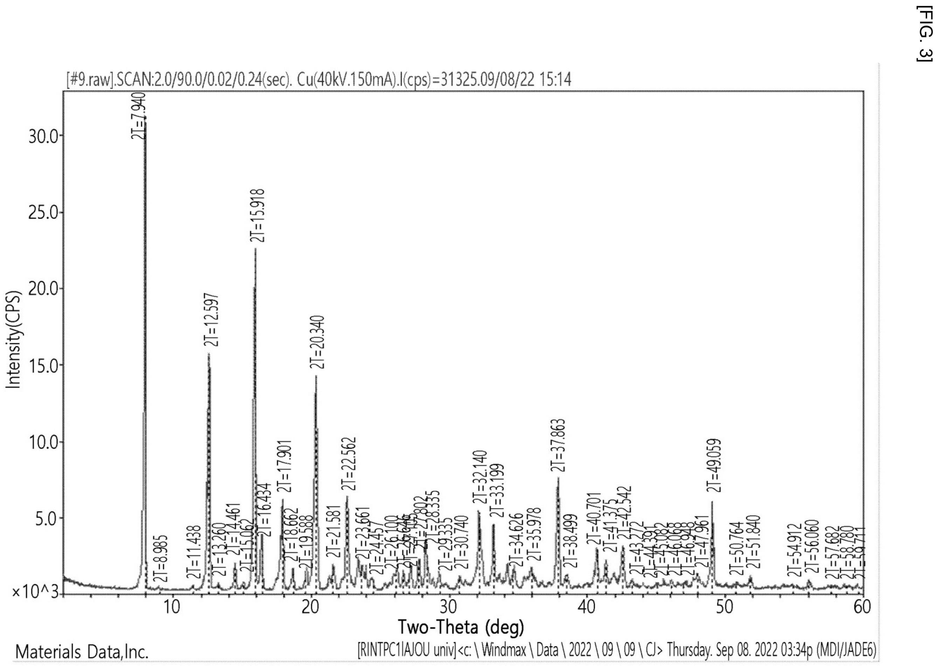

FIG. 3 shows the results of XRD analysis of disodium-5'-guanylate crystals obtained according to Example 2.

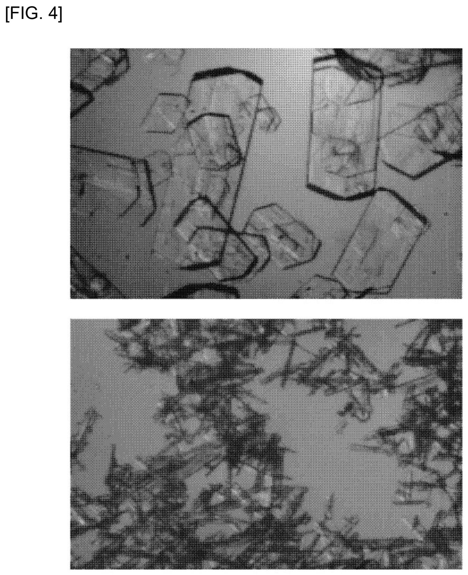

FIG. 4 is an image of disodium-5'-guanylate crystals obtained according to Example 4 observed under a microscope.

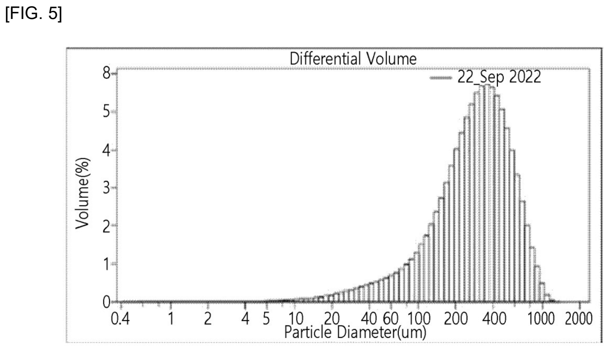

FIG. 5 shows the particle size analysis result of disodium-5'-guanylate crystals obtained according to Example 4.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

The present disclosure relates to a method for preparing disodium-5'-guanylate crystals, and the formation of amorphous crystals of disodium-5'-guanylate and the transition of amorphous crystals into columnar crystals may be performed continuously by concentrating the mixed solution of aqueous disodium-5'-guanylate solution and aqueous sodium chloride solution and adding seeds thereto. Accordingly, disodium-5'-guanylate crystals can be prepared with high yield in an environmentally friendly manner.

FIG. 1 is a flow chart showing the method for preparing disodium-5'-guanylate according to the present disclosure.

With reference to FIG. 1, the method for preparing disodium-5'-guanylate crystals according to the present disclosure includes: a mixing step **(S100)** of adding an aqueous sodium chloride solution to an aqueous disodium-5'-guanylate solution;
a concentration step **(S200)** of concentrating the mixed solution of the aqueous disodium-5'-guanylate solution and the aqueous sodium chloride solution, and precipitating amorphous crystals of disodium-5'-guanylate; and
a transition step **(S300)** of adding seeds to the mixed solution of the aqueous disodium-5'-guanylate solution and the aqueous sodium chloride solution to transform the amorphous crystals of disodium-5'-guanylate into columnar crystals. Hereinafter, each step of the method for preparing disodium-5'-guanylate crystals of the present disclosure will be examined.

First, in the mixing step **(S100),** an aqueous sodium chloride solution is added to an aqueous disodium-5'-guanylate solution and mixed.

The aqueous disodium-5'-guanylate solution used in the mixing step **(S100)** may be a fermented product prepared by fermentation. As used herein, the "fermented product" may refer to a product resulting from enzymatic or metabolic decomposition of organic substances using microorganisms. For example, the fermented product may include the culture itself obtained by culturing a microorganism in a culture medium, or a concentrate, dried product, or freeze-dried product of the culture obtained by removing bacterial cells therefrom. In addition, in this case, a fermentation liquid may include the entire fermented product including disodium-5'-guanylate, or may be one in which impurities have been removed from the fermented product including disodium-5'-guanylate.

The "microorganism for producing disodium-5'-guanylate" or "microorganism for producing disodium-5'-guanylate or a desired product" used in the mixing step **(S100)** may include all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired protein or disodium-5'-guanylate.

The microorganism for producing disodium-5'-guanylate of the present disclosure may be a microorganism naturally having a disodium-5'-guanylate-producing ability, or a microorganism, in which a disodium-5'-guanylate-producing ability has been imparted to a parent strain having no disodium-5'-guanylate-producing ability, but is not limited thereto. Specifically, as used herein, the microorganism for producing disodium-5'-guanylate or a desired product, or the microorganism having a disodium-5'-guanylate- or desired product-producing ability may be a microorganism in which some genes in the biosynthesis pathway of the desired protein or desired product are enhanced or weakened, or some genes in the degradation pathway of the desired protein or desired product are enhanced or weakened. The "enhancement" or "increase" in the disodium-5'-guanylate-producing ability of the microorganism of the present disclosure may mean that the disodium-5'-guanylate-producing ability of the microorganism of the present disclosure is enhanced compared to that of a microorganism other than the microorganism of the present disclosure, a parent strain, or a non-modified microorganism. In one example, the microorganism of the present disclosure may have an enhanced disodium-5'-guanylate-producing ability by about 1% or more, 10% or more, 100% or more 200% or more 500% or more 1000% or more, 1100% or more, 1200% or more, 1300% or more, or about 1.01 times or more, 2 times or more, 5 times or more, 10 times or more, 11 times or more, 12 times or more, or 13 times or more compared to the disodium-5'-guanylate-producing ability of other microorganisms, but is not limited thereto. As used herein, the term "about" refers to a range including all of ±0.5, ±0.4, ±0.3, ±0.2, ±0.1, *etc.,* and it includes all of the values equivalent to those which come immediately after the term "about" or those in a similar range, but is not limited thereto.

The above-mentioned microorganism used in the mixing step **(S100)** may be at least one selected from the group consisting of *Candida famata,* which is a yeast, *Eremothecium ashbyii* and *Ashbyagossypii,* which are ascomycetes, *Bacillus subtilis* and microorganisms belonging to the genus *Corynebacterium,* which are bacteria.

When the microorganism used in the mixing step **(S100)** is a microorganism belonging to the genus *Corynebacterium,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* more specifically, *Corynebacterium glutamicum,* but is not limited thereto.

The mixing step may further include culturing "the microorganism for producing disodium-5'-guanylate". The culturing of the microorganism may be performed in a suitable culture medium and culture conditions known in the art. Such a culturing process may be easily adjusted for use by those skilled in the art according to the strain to be selected. Specifically, the culturing may be a batch culture, a continuous culture, and/or a fed-batch culture, but is not limited thereto. As used herein, the term "medium" refers to a mixture of materials which contains nutrient materials required for culturing of the microorganism as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

The aqueous disodium-5'-guanylate solution used in the mixing step **(S100)** may be prepared by dissolving a fermented product including disodium-5'-guanylate prepared by the above-described process in an aqueous solvent. For example, the aqueous disodium-5'-guanylate solution may be prepared by dissolving disodium-5'-guanylate in crystal form obtained from the fermented product in water. In the aqueous disodium-5'-guanylate solution, disodium-5'-guanylate may exist in the form of a hydrate. For example, disodium-5'-guanylate may be provided in the form of disodium-5'-guanylate heptahydrate or disodium-5'-guanylate tetrahydrate. In the present disclosure, an aqueous solvent is used, and an organic solvent is not used. Accordingly, the preparation method of the present disclosure may be performed in an environmentally friendly manner as the use of organic solvents is excluded.

The aqueous disodium-5'-guanylate solution used in the mixing step **(S100)** may have a disodium-5'-guanylate concentration of about 50 g/L to about 600 g/L. In some cases, the disodium-5'-guanylate concentration may be about 50 g/L to about 550 g/L, about 50 g/L to about 500 g/L, about 50 g/L to about 450 g/L, about 50 g/L to about 400 g/L, about 50 g/L to about 350 g/L, about 50 g/L to about 300 g/L, about 50 g/L to about 250 g/L, about 50 g/L to about 200 g/L, about 50 g/L to about 150 g/L, about 50 g/L to about 100 g/L, about 100 g/L to about 450 g/L, about 150 g/L to about 450 g/L, about 200 g/L to about 450 g/L, about 250 g/L to about 450 g/L, about 300 g/L to about 450 g/L, about 350 g/L to about 450 g/L, and about 400 g/L to about 450 g/L. By preparing an aqueous solution of disodium-5'-guanylate in the above-mentioned concentration range, amorphous crystals of disodium-5'-guanylate may be sufficiently produced when the aqueous disodium-5'-guanylate solution is prepared in the above-mentioned concentration range and mixed with sodium chloride.

In the mixing step **(S100),** the aqueous sodium chloride solution is mixed with the aqueous disodium-5'-guanylate solution prepared as described above. The aqueous sodium chloride solution may be a solution in which sodium chloride (NaCl) is homogeneously dissolved in an aqueous solvent. The aqueous solvent used to prepare the aqueous sodium chloride solution and the aqueous solvent used to prepare the aqueous disodium-5'-guanylate solution described above may be the same or different types. For example, both aqueous solutions can be prepared using water as a solvent. In the present disclosure, as discussed above, organic solvents are not used in the process of preparing disodium-5'-guanylate crystals. According to the existing technology, the solubility of disodium-5'-guanylate was reduced by treatment with an organic solvent to precipitate crystals. When a hydrophilic solvent is used instead of an organic solvent, an additive that can lower the solubility of the solvent is required. According to the present disclosure, sodium chloride, which is approved as a food additive, is added to lower the solubility of disodium-5'-guanylate in the hydrophilic solvent.

The aqueous sodium chloride solution used in the mixing step **(S100)** may be in a state before supersaturation in which sodium chloride salt has not precipitated. The aqueous sodium chloride solution may be a solution with a sodium chloride concentration of about 10 g/L to 300 g/L. In some cases, the concentration of sodium chloride may be about 10 g/L to 200 g/L, about 10 g/L to 100 g/L, about 50 g/L to 300 g/L, about 100 g/L to 300 g/L, and about 200 g/L to 300 g/L. The concentration of the aqueous sodium chloride solution can be determined in consideration of the concentration and amount of the aqueous disodium-5'-guanylate solution used in the mixing step **(S100).**

In the mixing step **(S100),** sodium chloride may be added at a ratio of 30% to 80% by weight of disodium-5'-guanylate. In some cases, sodium chloride may be added at a ratio of about 40% to about 70% or about 40% to about 60% by weight of disodium-5'-guanylate. This is different from the technology that causes phase transition by treating sodium chloride to a level of 90 to 120% by weight of disodium-5'-guanylate. The method of adding excessive amounts of sodium chloride increases the cost as the amount of salt used increases, and when the salt concentration rises to the point of supersaturation of the salt during the subsequent concentration process, there is a risk of a decrease in content as NaCl is precipitated, and the crystals may be formed in fine particles. In comparison, according to one aspect of the present disclosure, the small amount of sodium chloride is used, and the salt in the solution is not supersaturated and thus is not precipitated, therefore, disodium-5'-guanylate crystals can be obtained in high yield.

There is no limitation to the method of mixing the aqueous disodium-5'-guanylate solution and the aqueous sodium chloride solution in the mixing step **(S100).** For example, the aqueous disodium-5'-guanylate solution and the aqueous sodium chloride solution can be mixed in various ways, such as adding the aqueous sodium chloride solution dropwise to the aqueous disodium-5'-guanylate solution, *etc.*

Next, a concentration step **(S200),** in which the mixed solution of disodium-5'-guanylate and sodium chloride is concentrated and the amorphous crystals of disodium-5'-guanylate are precipitated, is performed.

In the concentration step **(S200),** the amorphous crystals of disodium-5'-guanylate are precipitated. The disodium-5'-guanylate can be provided in the form of a hydrate when mixed with the aqueous sodium chloride solution, and disodium-5'-guanylate in hydrate form forms nuclei when the solubility reaches the point of supersaturation. Next, the nuclei of disodium-5'-guanylate gather together and amorphous crystals are precipitated.

The concentration step **(S200)** is performed to increase the concentration of disodium-5'-guanylate. The concentration step **(S200)** may be performed so that the disodium-5'-guanylate is concentrated to a concentration of 400 g/L to 600 g/L. In some cases, in the concentration step **(S200),** the disodium-5'-guanylate may be concentrated to a concentration of 400 g/L to 550 g/L, 400 g/L to 500 g/L, 400 g/L to 450 g/L, 450 g/L to 600 g/L, 500 g/L to 600 g/L, and 550 g/L to 600 g/L. By concentrating the mixed solution to the above-mentioned concentration range, a phase transition continuously occurs as the seeds are added in the transition step **(S300),** thus, the amorphous crystals, which are continuously generated during the concentration process, are formed into tetrahydrate or heptahydrate columnar crystals.

There is no limitation to the method of performing the concentration step **(S200).** Concentration may be performed using a conventional concentrator (*e.g.,* forced circulation concentrator, thin film concentrator, or rotary concentrator, *etc.*) according to the selection of a person skilled in the art.

The concentration step **(S200)** may be performed for about 2 hours to about 15 hours. In some cases, the concentration may be performed for about 2 hours to about 10 hours, about 2 hours to about 5 hours, about 5 hours to about 15 hours, or about 10 hours to about 15 hours. The time of performing the concentration can be determined in consideration of disodium-5'-guanylate concentration, the production amount of disodium-5'-guanylate, *etc.*

In the concentration step **(S200),** the temperature inside the concentrator may be adjusted to a range from about 25°C to about 85°C. In some cases, the temperature inside the concentrator may be adjusted to a range from about 25°C to about 75°C, about 25°C to about 65°C, about 25°C to about 55°C, about 25°C to about 45°C, about 25°C to about 35°C, about 35°C to about 85°C, about 35°C to about 75°C, about 35°C to about 65°C, about 35°C to about 55°C, about 35°C to about 45°C, about 45°C to about 85°C, about 45°C to about 75°C, about 45°C to about 65°C, about 45°C to about 55°C, about 55°C to about 85°C, about 55°C to about 75°C, about 55°C to about 65°C, about 65°C to about 85°C, about 65°C to about 75°C, or about 75°C to about 85°C. The disodium-5'-guanylate crystals may be obtained without denaturation of disodium-5'-guanylate to be produced by maintaining the temperature range of the concentration process within the above-mentioned range. If necessary, the degree of vacuum inside the concentration device may be adjusted so that the temperature does not change outside the above-mentioned range while performing concentration.

During the concentration step **(S200),** a transition step **(S300),** in which seeds are added to transform the amorphous crystals of disodium-5'-guanylate into columnar crystals, is performed. In particular, performing the transition step **(S300)** while performing the concentration step **(S200)** may mean concentrating the solution so that the concentration of the mixed solution reaches a certain level and then adding seeds. However, while adding seeds, the concentration of disodium-5'-guanylate in the solution may be maintained within a certain range by continuously concentrating the solution as needed. Additionally, in some cases, if the concentration of disodium-5'-guanylate in the solution provided after mixing in the mixing step **(S100)** is at a level at which amorphous crystals can be precipitated, a transition step of adding seeds may be performed at the same time as the start of the concentration step **(S200).** Accordingly, the concentration step **(S200)** does not necessarily have to be performed prior to the transition step **(S300).**

The seeds added in the transition step **(S300)** may be crystals of disodium-5'-guanylate. After seed injection, the amorphous crystals of disodium-5'-guanylate may be transformed into columnar crystals. The columnar crystals have a clearer shape and higher crystallinity compared to amorphous crystals. Therefore, the columnar crystals can be relatively easily separated from the mother liquid, thereby increasing the yield of disodium-5'-guanylate. The columnar crystals of disodium-5'-guanylate may include disodium-5'-guanylate in the form of disodium-5'-guanylate heptahydrate.

In the transition step **(S300),** the seeds can be added when the concentration of sodium chloride in the mixed solution is at the level of about 100 g/L to about 130 g/L. As the concentration is continued even after seed addition, the formation of amorphous crystals of disodium-5'-guanylate and the transition to columnar crystals of disodium-5'-guanylate according to seed addition may occur simultaneously.

In the transition step **(S300),** the size of the columnar crystals of disodium-5'-guanylate formed may vary depending on the condition of seed injection. Specifically, as previously examined, if the seeds are added when the concentration of sodium chloride in the mixed solution is about 100 g/L to about 130 g/L, the formation of amorphous crystals of disodium-5'-guanylate and the transition to columnar crystals of disodium-5'-guanylate according to seed addition may occur simultaneously, and disodium-5'-guanylate crystals having excellent crystallinity may be obtained.

In contrast, when the seeds are added outside the above-mentioned concentration range, in particular, when the seeds are added as the concentration of sodium chloride is higher than the above-mentioned range, the transition speed of the crystals may slow down and the crystals may be formed in fine particles rather than a columnar form. In order to obtain columnar crystals in high yield in the transition step **(S300),** it is important to adjust process factors such as the concentration of disodium-5'-guanylate in the solution, the temperature of the solution, and the concentration of sodium chloride, as previously examined. This is because various factors such as crystallization solvent, temperature, concentration of disodium-5'-guanylate, cation concentration, other derivatives, *etc*. may serve as a factor that affects the crystal phase transition process, and interfere with the phase transition. If any of the elements that create the crystallization environment are processed incorrectly, the phase transition of the crystal may not occur smoothly, and even if the phase transition occurs, it may be obtained in the form of small fine crystals, which may reduce the process yield.

The temperature for performing the transition step **(S300)** and the concentration step **(S200)** may be varied in consideration of the form of disodium-5'-guanylate. For example, when the disodium-5'-guanylate is provided in the form of disodium-5'-guanylate heptahydrate, the process temperature may be adjusted to a range of about 25°C to about 42°C to efficiently induce the formation of amorphous crystals by concentration and the transition into columnar crystals. Additionally, when the disodium-5'-guanylate is provided in the form of disodium-5'-guanylate tetrahydrate, the process temperature may be adjusted to a range of about 42°C to about 85°C to efficiently induce the formation of amorphous crystals by concentration and the transition into columnar crystals.

The columnar crystals of disodium-5'-guanylate, which is the final product, may be obtained by separating the columnar crystals of disodium-5'-guanylate formed after performing the transition step **(S300)** from the mother liquid. In order to separate crystals, various methods such as reduced pressure membrane filtration device, pressure-driven membrane filtration device, centrifugal separation device, basket separation, *etc*. may be used.

As described above, according to the present disclosure, disodium-5'-guanylate crystals are prepared using an aqueous solvent without an organic solvent, and thus are environmentally friendly. Additionally, the yield of disodium-5'-guanylate crystals may be increased by adjusting the process factors so that disodium-5'-guanylate are prepared in the form of amorphous crystals and then undergoes a phase transition to columnar crystals.

In the above, the method for preparing disodium-5'-guanylate crystals according to one aspect of the present disclosure was examined. Hereinbelow, more specific aspects of the method for preparing disodium-5'-guanylate will be examined.

FIG. 2 is a flowchart showing the method for preparing disodium-5'-guanylate crystals according to one aspect of the present disclosure.

In describing FIG. 2, description that overlaps with those previously described in FIG. 1 will be omitted to avoid duplication of content.

With reference to FIG. 2, in the mixing step **(S100),** the aqueous disodium-5'-guanylate solution may be prepared by dissolving disodium-5'-guanylate wet crude crystals prepared by fermentation. Dissolution of disodium-5'-guanylate wet crude crystals maybe be performed by a conventional method, such as adding disodium-5'-guanylate wet crude crystals to an aqueous solvent and then stirring, *etc.*

Additionally, before performing the mixing step **(S100),** a step of adjusting the pH of the aqueous disodium-5'-guanylate solution may be performed. Specifically, the pH of the aqueous disodium-5'-guanylate solution may be adjusted to 8 to 10. In the above-mentioned pH range, the transition of disodium-5'-guanylate into columnar crystals may be rapidly carried out. The pH adjustment of the aqueous disodium-5'-guanylate solution may be performed by adding sodium salt. Therefore, it is possible to prevent cations other than sodium ions (Na⁺) from being added to the mixed solution and interfering with crystallization of disodium-5'-guanylate. Examples of sodium salts that can be used to adjust pH may include sodium hydroxide, monosodium citrate, disodium citrate, trisodium citrate, monosodium phosphate, disodium phosphate, trisodium phosphate, sodium carbonate, sodium bicarbonate, *etc.*

In the mixing step **(S100),** a decolorization process may be additionally performed to remove impurities. The decolorization process is for remove residual coloring substances in the fermentation liquid, and may be performed by an adsorption decolorization method using activated carbon or white clay (activated clay, acidic clay) as an adsorbent. However, the above-mentioned description is for illustrative purposes, and the method of performing the decolorization process may be appropriately selected considering the physical properties of the fermentation liquid containing L-citrulline and the characteristics of the subsequent processes, *etc*. The order of pH adjustment, bacterial filtration, and decolorization process may vary as needed. For example, the pH may be adjusted after completing bacterial filtration and decolorization, or decolorization may be performed first before bacterial cell filtration.

After the columnar crystals of disodium-5'-guanylate are formed following the transition step **(S300),** steps of cooling the solution, separating the columnar crystals, and drying the separated columnar crystals may be performed. Drying of the crystals may be performed at room temperature.

According to one aspect of the present disclosure, the columnar crystals of disodium-5'-guanylate prepared by the above-described method may be provided in the form of a mixed product with disodium-5'-inosinate crystals. Specifically, according to one aspect of the present disclosure, there may be provided a fermentation product, which includes disodium-5'-guanylate crystals in columnar form; and disodium 5'-inosinate crystals, wherein the concentration of chlorine ions (Cl⁻) in the mixture of disodium-5'-guanylate crystals and disodium-5'-inosinate crystals is 500 ppm to 20,000 ppm.

Additionally, according to one aspect of the present disclosure, there may be provided a crystal or a fermentation product, which includes disodium-5'-guanylate crystals, wherein the disodium-5'-guanylate crystals have a chloride ion concentration of 500 ppm to 20,000 ppm.

The disodium-5'-guanylate crystals according to one aspect of the present disclosure may have a chlorine ion (Cl⁻) concentration of 500 ppm to 20,000 ppm within the crystals. The disodium-5'-guanylate crystals having a chlorine ion concentration in the above-mentioned range may be provided in the form of columnar crystals. Since the disodium-5'-guanylate crystals in the form of columnar crystals can be easily separated during the preparation process, they may contain a small amount of impurities and may be provided with high crystal purity.

Additionally, when the chlorine ion concentration is in the above-mentioned range, microbial growth can be controlled, and solidification due to the environment, in which the manufactured product is distributed or stored, can be prevented. Acorcdingly, the disodium-5'-guanylate crystals containing the above-mentioned chlorine ion concentration not only have high purity, but are also advantageous in terms of storage and distribution. In addition, when the chlorine ions remain in the final products at a concentration of 500 ppm to 20,000 ppm, the microbial growth may be controlled and solidification due to pressure may be reduced, which enables to easily manage the products, and the contamination rate by microorganisms may decrease, thus, the products can be safely consumed.

The disodium-5'-guanylate having the above-described chlorine ion concentration may be prepared by the method for preparing disodium-5'-guanylate crystals according to one aspect of the present disclosure examined above. According to the method for preparing disodium-5'-guanylate crystals according to one aspect of the present disclosure, the formation of amorphous crystals of disodium-5'-guanylate and the transition to columnar crystals of disodium-5'-guanylate according to seed addition may occur simultaneously as the seeds are added when the concentration of sodium chloride in the mixed solution is the concentration of disodium-5'-guanylate is at the level of about 100 g/L to about 130 g/L. The disodium-5'-guanylate crystals prepared according to such a preparation method may contain a specific concentration of chlorine ions within the crystal because sodium chloride is added at a specific concentration in the transition step. Specifically, chlorine ions are provided at a concentration of 500 ppm to 20,000 ppm within the crystals.

In comparison, it was confirmed through experiments that less than 100 ppm of chlorine ions were provided in the disodium-5'-guanylate crystals prepared by a method according to the prior art other than the method according to the present disclosure, e.g., an ethanol crystallization method.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

In the above, the method for preparing disodium-5'-guanylate crystals according to one aspect of the present disclosure was examined. Hereinafter, the beneficial effects mentioned in the present disclosure will be described through Example and Comparative Example experimental results.

### Comparative Example 1. Crystallization using Hydrophilic Organic Solvent (Conventional Crystallization Method)

0.5 L of a 30 wt.% disodium-5'-guanylate solution was prepared by dissolving crude disodium-5'-guanylate crystals of 90% purity derived from microbial fermentation liquid. Afterwards, 0.55 L of ethanol was added at room temperature to form amorphous crystals. While adding ethanol, 10 wt.% of disodium-5'-guanylate crystal heptahydrate seeds were added by weight, and then ethanol was continuously added to induce a phase transition from disodium-5'-guanylate amorphous crystals into disodium-5'-guanylate heptahydrate crystals. After cooling the crystal slurry to an internal temperature of 25°C, the crystals were separated using a centrifuge and dried at room temperature to obtain a product. The quality of the obtained product showed the following: a content of 100%, a moisture of 23%, and a yield of 95%. The disodium-5'-guanylate heptahydrate crystals were slurried using 99.5% ethanol and subjected to wet particle size analysis using a particle size analyzer. The average particle size was at the level of 150 µm. The Cl⁻ residual amount in the final crystal analyzed by chromatography was at the level of 39.2 ppm.

### Comparative Example 2. Non-concentrated Aqueous Crystallization

0.5 L of a 30 wt.% disodium-5'-guanylate solution was prepared by dissolving crude disodium-5'-guanylate crystals. 0.55 L of a 30 wt.% solution was prepared by dissolving sodium chloride in water. The amount of sodium chloride relative to the disodium-5'-guanylate solution was about 90% by mass. At a temperature of about 42°C, the sodium chloride solution was added dropwise to the disodium-5'-guanylate solution to induce a phase transition from disodium-5'-guanylate amorphous crystals to disodium-5'-guanylate heptahydrate crystals. After slowly cooling to about 25°C, the separated crystals using a basket centrifuge had a content of 98%, moisture of 25%, and yield of 69%.

### Comparative Example 3. Non-concentrated Aqueous Crystallization

0.2 L of a 30 wt.% disodium-5'-guanylate solution was prepared by dissolving crude disodium-5'-guanylate crystals and then 50 wt.% NaOH (sodium hydroxide solution) was added thereto to adjust the pH to 9.0. 0.2 L of a 30 wt.% solution was prepared by dissolving sodium chloride in water. The amount of sodium chloride relative to the disodium-5'-guanylate solution was about 40% by mass. The temperature of the homogeneous solution from which the amorphous crystals had been precipitated by mixing the two solutions was measured with a thermometer and maintained at a temperature not exceeding about 25°C to about 42°C. 1 wt.% of disodium-5'-guanylate heptahydrate seeds were added to induce a phase transition, but no phase transition occurred. The amorphous crystals were centrifuged using a basket separator, but the crystals could not be separated.

### Experimental Example 1 - Synthesis of Disodium-5'-Guanylate according to Amount of Sodium Chloride Added

### Example 1

0.5 L of a 30 wt.% disodium-5'-guanylate solution was prepared by dissolving crude disodium-5'-guanylate crystals and then 50 wt.% NaOH (sodium hydroxide solution) was added thereto to adjust the pH to 9.0. 0.2 L of a 30 wt.% solution was prepared by dissolving sodium chloride in water. The amount of sodium chloride relative to the mass of disodium-5'-guanylate in the disodium-5'-guanylate solution was about 40% by mass. Concentration crystallization was initiated in a homogeneous solution from which the amorphous crystals had been precipitated by mixing the two solutions. Concentration was carried out by adjusting the degree of vacuum and measuring the internal temperature with a thermometer such that the temperature did not exceed 25°C to 42°C. At a concentration level of 2.1-fold, 1 wt.% of disodium-5'-guanylate heptahydrate seeds were added to induce a phase transition continuously. After seed injection, the amorphous crystals of disodium-5'-guanylate were continuously transformed into disodium-5'-guanylate heptahydrate crystals in columnar form. During the phase transition, the disodium-5'-guanylate heptahydrate was concentrated until the concentration reached at least 48 wt.%. The concentrated crystal slurry was cooled to an internal temperature of 25°C, and the crystals were separated using a centrifuge and dried at room temperature. The obtained disodium-5'-guanylate crystals had a content of 100%, moisture of 23%, and yield of 93%. The disodium-5'-guanylate heptahydrate crystals were slurried using 99.5% ethanol and subjected to wet particle size analysis using a particle size analyzer. As a result, the average particle size was at the level of 250 µm. The amount of residual anions (Cl⁻) in the final crystals analyzed by HPLC was at the level of 1474.9 ppm.

### Example 2

0.5 L of a 30 wt.% disodium-5'-guanylate solution was prepared by dissolving crude disodium-5'-guanylate crystals and then 50 wt.% NaOH (sodium hydroxide solution) was added thereto to adjust the pH to 9.0. Thereafter, sodium carbonate anhydrous was added thereto at a ratio of 5% by weight of disodium-5'-guanylate. 0.33 L of a 30 wt.% solution was prepared by dissolving sodium chloride in water. The amount of sodium chloride relative to the mass of disodium-5'-guanylate in the disodium-5'-guanylate solution was about 60% by mass. Concentration crystallization was initiated in a homogeneous solution from which the amorphous crystals had been precipitated by mixing the two solutions. Concentration was carried out by measuring the internal temperature with a thermometer such that the temperature did not exceed 25°C to 42°C. At a concentration level of 1.05-fold, 1 wt.% of disodium-5'-guanylate heptahydrate seeds were added to induce a phase transition continuously. After seed injection, the amorphous crystals of disodium-5'-guanylate were continuously transformed into disodium-5'-guanylate heptahydrate crystals in columnar form, and the disodium-5'-guanylate heptahydrate was concentrated until the concentration reached at least 44 wt.%. The concentrated crystal slurry was cooled to an internal temperature of 25°C, and the crystals were separated using a centrifuge and dried at room temperature. The obtained disodium-5'-guanylate crystals had a content of 100.7%, moisture of 23%, and yield of 85%. The disodium-5'-guanylate heptahydrate crystals were slurried using 99.5% ethanol and subjected to wet particle size analysis using a particle size analyzer. As a result, the average particle size was at the level of 290 µm. The amount of residual anions (Cl⁻) in the final crystals analyzed by HPLC was at the level of 4723 ppm.

FIG. 3 shows the results of XRD analysis of disodium-5'-guanylate crystals obtained according to Example 2.

When the peaks appearing as a result of the XRD analysis were analyzed, the results were confirmed to be consistent with the XRD values shown in the columnar form of disodium-5'-guanylate heptahydrate previously reported in the literature. Accordingly, it was confirmed from the results of FIG. 3 that columnar form of disodium-5'-guanylate heptahydrate crystals were synthesized according to one aspect of the present disclosure.

### Example 3

0.5 L of a 30 wt.% disodium-5'-guanylate solution was prepared by dissolving crude disodium-5'-guanylate crystals and then sodium carbonate anhydrous was added thereto at a ratio of 5% by weight of disodium-5'-guanylate. 0.25 L of a 30 wt.% solution was prepared by dissolving sodium chloride in water. The amount of sodium chloride relative to the mass of disodium-5'-guanylate in the disodium-5'-guanylate solution was about 50% by mass. Concentration crystallization was initiated in a homogeneous solution from which the amorphous crystals had been precipitated by mixing the two solutions. Concentration was carried out by measuring the internal temperature with a thermometer such that the temperature did not exceed 25°C to 42°C. At a concentration level of 1.39-fold, 1 wt.% of disodium-5'-guanylate heptahydrate seeds were added to induce a phase transition continuously. After seed injection, the amorphous crystals of disodium-5'-guanylate were continuously transformed into disodium-5'-guanylate heptahydrate crystals in columnar form, and the disodium-5'-guanylate heptahydrate was concentrated until the concentration reached at least 45 wt.%. The concentrated crystal slurry was cooled to an internal temperature of 25°C, and the crystals were separated using a centrifuge and dried at room temperature. The obtained disodium-5'-guanylate crystals had a content of 100.4%, moisture of 23%, and yield of 87%. The disodium-5'-guanylate heptahydrate crystals were slurried using 99.5% ethanol and subjected to wet particle size analysis using a particle size analyzer. As a result, the average particle size was at the level of 397 µm. The amount of residual anions (Cl⁻) in the final crystals analyzed by HPLC was at the level of 2660 ppm.

### Example 4

0.5 L of a 30 wt.% disodium-5'-guanylate solution was prepared by dissolving crude disodium-5'-guanylate crystals and then 50 wt.% NaOH (sodium hydroxide solution) was added thereto to adjust the pH to 9.0. 0.3 L of a 30 wt.% solution was prepared by dissolving sodium chloride in water. The amount of sodium chloride relative to the disodium-5'-guanylate solution was about 60% by mass. Concentration crystallization was initiated in a homogeneous solution from which the amorphous crystals had been precipitated by mixing the two solutions. Concentration was carried out by adjusting the degree of vacuum and measuring the internal temperature with a thermometer such that the temperature did not exceed 25°C to 42°C. At a concentration level of 1.12-fold, 1 wt.% of disodium-5'-guanylate heptahydrate seeds were added to induce a phase transition continuously. After seed injection, the amorphous crystals of disodium-5'-guanylate were continuously transformed into disodium-5'-guanylate heptahydrate crystals in columnar form, and the disodium-5'-guanylate heptahydrate was concentrated until the concentration reached at least 44 wt.%. The concentrated crystal slurry was cooled to an internal temperature of 25°C, and the crystals were separated using a centrifuge and dried at room temperature. The obtained disodium-5'-guanylate crystals had a content of 102%, moisture of 22.5%, and yield of 88%. The disodium-5'-guanylate heptahydrate crystals were slurried using 99.5% ethanol and subjected to wet particle size analysis using a particle size analyzer. As a result, the average particle size was at the level of 329 µm. The amount of residual anions (Cl⁻) in the final crystals analyzed by HPLC was at the level of 1874 ppm (FIG. 2).

### Example 5

0.5 L of a 30 wt.% disodium-5'-guanylate solution was prepared by dissolving crude disodium-5'-guanylate crystals and then 50 wt.% NaOH (sodium hydroxide solution) was added thereto to adjust the pH to 9.0. 0.25 L of a 30 wt.% solution was prepared by dissolving sodium chloride in water. The amount of sodium chloride relative to the mass of disodium-5'-guanylate in the disodium-5'-guanylate solution was about 50% by mass. Concentration crystallization was initiated in a homogeneous solution from which the amorphous crystals had been precipitated by mixing the two solutions. Concentration was carried out by adjusting the degree of vacuum and measuring the internal temperature with a thermometer such that the temperature did not exceed 25°C to 42°C. At a concentration level of 1.4-fold, 1 wt.% of disodium-5'-guanylate heptahydrate seeds were added to induce a phase transition continuously. After seed injection, the amorphous crystals of disodium-5'-guanylate were continuously transformed into disodium-5'-guanylate heptahydrate crystals in thin columnar form, and the disodium-5'-guanylate heptahydrate was concentrated until the concentration reached at least 40 wt.%. The concentrated crystal slurry was cooled to an internal temperature of 25°C, and the crystals were separated using a centrifuge and dried at room temperature. The obtained disodium-5'-guanylate crystals had a content of 111.5%, moisture of 17.6%, and yield of 88.4%. The disodium-5'-guanylate heptahydrate crystals were slurried using 99.5% ethanol and subjected to wet particle size analysis using a particle size analyzer. As a result, the average particle size was at the level of 130 µm. The amount of residual anions (Cl⁻) in the final crystals analyzed by HPLC was at the level of 2478 ppm.

FIG. 4 is an image of disodium-5'-guanylate crystals obtained according to Example 4 observed under a microscope. As can be seen from the drawing, the obtained crystals were in columnar form, and no amorphous crystals were observed. Therefore, it was confirmed that most of the disodium-5'-guanylate in the solution was obtained in the form of columnar crystals through continuous phase transition of the crystals, and thus disodium-5'-guanylate crystals could be obtained in high yield.

FIG. 5 shows the particle size analysis result of disodium-5'-guanylate crystals obtained according to Example 4, and it can be confirmed that the particle size of the crystals is relatively uniformly distributed.

### Experimental Example 2. Examination of Microbial Growth and Solidification Phenomenon according to Chlorine Ion Concentration in Disodium-5'-Guanylate Crystals

### (1) Confirmation of Microbial Growth Control Effect

The amounts of residual chlorine ions in the crystals prepared by the preparation process of Comparative Example 1 (Hydrophilic Organic Solvent Crystallization) and the crystals prepared by the preparation process of Example 1 are as follows.

**[Table 1]**

| | Residual amount of Cl ions (ppm) |
|---|---|
| Crystals prepared by the method of Comparative Example 1 | 34 |
| Crystals prepared by the method of Example 1 | 5, 703 |
| Crystals prepared by the method of Example 1 | 15,388 |
| Crystals prepared by the method of Example 1 | 20,000 |

As a result of comparing the microbial reduction rate after applying microorganisms to the crystals, it was confirmed that the microbial growth control effect was observed in the crystals prepared by the preparation method according to Example, which contained a chlorine ion concentration at the level of 5,703 ppm to 20,000 ppm. In particular, in the case of crystals containing a chlorine ion concentration of 20,000 ppm, it was confirmed that the microbial reduction effect was similar to the microbial proliferation control effect of salt. In contrast, the amount of residual chlorine in the crystals prepared by the hydrophilic organic solvent method (Comparative Example 1) was at the level of 34 ppm, and the microorganisms tended to proliferate in large quantities over time. The microbial growth control rate over time for each crystal is as follows.

**[Table 2]**

| | Residual amount of Cl ions (ppm) | number of microbial cells | | | | microbial reduction rate (by time) | | |
|---|---|---|---|---|---|---|---|---|
| | | initial value | 1 hour | 2 hours | 4 hours | 1 hour | 2 hours | 4 hours |
| Crystals prepared by the method of Comparative Example 1 | 34 | 60,000 | 110,000 | 50,000 | 60,000 | -83% | 17% | 0% |
| Crystals prepared by the method of Example 1 | 5,703 | 88,889 | 77,778 | 77,778 | 33,333 | 13% | 13% | 63% |
| Crystals prepared by the method of Example 1 | 15,388 | 100,000 | 66,667 | 50,000 | 33,333 | 33% | 50% | 67% |
| Crystals prepared by the method of Example 1 | 20,000 | 40,000 | 20,000 | 10,000 | 10,000 | 50% | 75% | 75% |
| salt | 603,448 | 160,000 | not measured | 30,000 | 20,000 | not measured | 81% | 88% |

### (2) Confirmation of Solidification Reduction Effect

Released products containing the prepared disodium-5'-guanylate may solidify due to the environment in which the products are distributed or stored. However, in the case of crystals containing chlorine, the solidification rate appears to be reduced, which is advantageous for storage compared to crystals prepared by the existing hydrophilic organic solvent method.

In order to confirm the reduction effect, the degree of solidification was measured using a ring shear tester. After injecting the crystals prepared by the preparation process of Comparative Example 1 (hydrophilic organic solvent crystallization) and the crystals prepared by the preparation process of Example 1 into the corresponding tester, SIGMA [Pa]: pressure value required to solidify the material; FC [Pa]: yield strength, pressure value required to break solidified material; and FFC: flowability were measured and the results are shown below.

**[Table 3]**

| | SIGMA [Pa] | FC [Pa] | FFC | Residual amount of Cl ions (ppm) |
|---|---|---|---|---|
| Crystals prepared by the method of Comparative Example 1 | 11,219 | 1.461 | 7.68 | 34 |
| Crystals prepared by the method of Example 1 | 11,469 | 1,129 | 10.16 | 445 |
| Crystals prepared by the method of Example 1 | 13.380 | 961 | 13.92 | 5,703 |
| Crystals prepared by the method of Example 1 | 12,941 | 1,282 | 10.09 | 15.388 |
| Crystals prepared by the method of Example 1 | 11, 189 | 1.094 | 10.23 | 20,000 |

The crystals (chlorine ion residual crystals) prepared by the method of Example 1 had a low FC value, which is the pressure value required to break the solidified material, and a high FFC value, proving that the crystals showed high flowability. Specifically, it was confirmed that the crystals having a chlorine ion residual amount of 5,703 ppm to 20,000 ppm prepared according to Example 1 had a FC value of 961 Pa to 1,129 Pa, which was lower than the FC value of 1,461 Pa obtained for the crystals having a chlorine ion residual amount of 34 ppm prepared according to Comparative Example 1. This means that the crystals having a high chlorine ion residual amount prepared according to Example were less solidified, and thus the pressure value to break the solidification was low.

Additionally, the crystals having a chlorine ion residual amount of 5,703 ppm to 20,000 ppm prepared according to Example 1 had a higher FFC value, which is an indicator of flowability, than the crystals having a chlorine ion residual amount of 34 ppm prepared according to Comparative Example 1, suggesting that solidification was reduced.

As such, it was confirmed that the disodium-5'-guanylate crystals prepared by the preparation method according to one embodiment of the present disclosure had a chlorine ion concentration in the range of 500 ppm to 20,000 ppm, and accordingly, solidification was reduced and microbial growth was controlled.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A method for preparing disodium-5'-guanylate crystals, comprising:
a mixing step of adding an aqueous sodium chloride solution to an aqueous disodium-5'-guanylate solution;
a concentration step of concentrating the mixed solution of the aqueous disodium-5'-guanylate solution and the aqueous sodium chloride solution, and precipitating amorphous crystals of disodium-5'-guanylate; and
a transition step of adding seeds to the mixed solution of the aqueous disodium-5'-guanylate solution and the aqueous sodium chloride solution to transform the amorphous crystals of disodium-5'-guanylate into columnar crystals.

2. The method of claim 1, wherein, in the transition step, the formation of amorphous crystals of disodium-5'-guanylate and the transition of amorphous crystals of disodium-5'-guanylate into columnar crystals are performed continuously.

3. The method of claim 1, wherein the amorphous crystals of disodium-5'-guanylate comprises a hydrate of disodium-5'-guanylate.

4. The method of claim 1, wherein the concentration step is performed such that the disodium-5'-guanylate is concentrated to a concentration of 400 g/L to 600 g/L.

5. The method of claim 1, further comprising adjusting the pH of the aqueous disodium-5'-guanylate solution to 8 to 10 before performing the mixing step.

6. The method of claim 5, wherein the pH adjustment of the aqueous disodium-5'-guanylate solution is performed by adding sodium salt.

7. The method of claim 1, wherein, in the mixing step, sodium chloride is added at a ratio of 30% to 80% by weight of disodium-5'-guanylate.

8. The method of claim 1, wherein, in the transition step, the seed is added at a ratio of 0.5% to 50% by weight of the disodium-5'-guanylate.

9. The method of claim 1, wherein, in the transition step, the seed is added when the concentration of disodium chloride in the mixed solution is the concentration of disodium-5'-guanylate is about 100 g/L to about 130 g/L.

10. A fermentation product, comprising disodium-5'-guanylate crystals in columnar form, wherein the concentration of chlorine ions (Cl⁻) in the disodium-5'-guanylate crystals is 500 ppm to 20,000 ppm.

11. The fermentation product of claim 10, further comprising disodium 5'-inosinate crystals, wherein the concentration of chlorine ions (Cl⁻) in the mixture of disodium-5'-guanylate crystals and disodium-5'-inosinate crystals is 500 ppm to 20,000 ppm.

12. The fermentation product of claim 10, wherein the disodium-5'-guanylate crystals are provided in the form of disodium-5'-guanylate heptahydrate or disodium-5'-guanylate tetrahydrate.
